# EUROPEAN PATENT SPECIFICATION

(11) **EP 3 501 588 B1**
(45) Date of publication and mention of the grant of the patent: **16.06.2021**
(21) Application number: 17841300.1
(22) Date of filing: 04.07.2017
(51) Int. Cl.: A61M 25/09

(54) **GUIDEWIRE**
FÜHRUNGSDRAHT
FIL-GUIDE

(30) Priority: 17.08.2016 JP 2016159989
(43) Date of publication of application: 26.06.2019
(73) Proprietor: Terumo Kabushiki Kaisha, Tokyo 151-0072 (JP)
(72) Inventor: INOUE, Shuhei, Fujinomiya-shi Shizuoka 418-0015 (JP)
(74) Representative: Casalonga
(86) International application number: PCT/JP2017/024562
(87) International publication number: WO 2018/034072

(56) References cited:
- WO-A2-03/041784
- JP-A- H02 104 370
- JP-A- H04 164 459
- JP-A- 2002 527 179
- JP-A- 2006 271 955
- US-A1- 2007 244 413
- US-A1- 2008 154 207
- US-A1- 2010 087 780
- US-A1- 2016 022 215
- US-B1- 6 290 656

## Description

### Technical Field

The present invention relates to a guide wire.

### Background Art

The guide wire is used for introducing and inducing a catheter used for treatment of a site in which a surgical operation is difficult, treatment for low invasion to a human body, examination of angiography in cardiac diseases and treatment thereof, and the like to a target site.

For example, a guide wire used for a percutaneous coronary intervention (PCI) is inserted together with a balloon catheter to a front side of a stenosis of a coronary artery which is the target site, in a state in which a distal end of the guide wire is caused to protrude from a distal end of the balloon catheter, under X-ray fluoroscopy. Next, the guide wire induces the distal portion of the balloon catheter to the vicinity of the stenosis.

It is preferable that the guide wire used in such an operation be excellent in flexibility and restorability, and torque transmission performance in which the torque from a hand portion is effectively transmitted to the distal portion. The characteristics of the guide wire are important for inducing a treatment device such as a balloon catheter to a target site in a complicatedly bent blood vessel.

Meanwhile, in some cases, the guide wire used for the PCI may perform the shaping at the distal portion of the core wire of the guide wire for the purpose of enhancing the blood vessel selectivity. It is known that the distal portion of the core wire is formed into a flat plate shape (see, for example, PTL 1) in order to facilitate the shaping. However, in the guide wire in which the distal portion of the core wire is formed in a flat plate shape, the rigidity of the distal portion of the core wire is lowered, and the torque transmission performance of the guide wire is lowered. Further, there is a limit to the length for making it possible to set the core wire in a flat plate shape, and there is a problem that the range that can be shaped is limited.

### Citation List

### Patent Literature

PTL 1: US Patent Application No. 2008/0234606

Other background prior art documents are the following:
US2007244413 (A1**)** discloses a medical guidewire having a core-to-tip construction that includes a core wire region surrounded by a flexible coil. The core wire having a distal tip segment that includes a proximal flat drop axially separated from a distal flat drop by a cylindrical or frusto-conical linking portion. The proximal and distal flat drops each having a pair of parallel planar surfaces, wherein the planar surfaces of the proximal flat drop are at an angle to the planar surfaces of the distal flat drop. The tip construction provides improved flexibility while maintaining columnar strength and providing excellent torsional characteristics.
WO03041784 (A2**)** discloses a guide wire for guiding a medical device within a patient is disclosed. The guide wire includes an elongate core with a flexible body disposed about and secured to a distal core section of the elongate core. A shapeable member extends distally from the distal core section, and the shapeable member preferably includes a non-rectangular, cross-sectional shape including a D-shape, semicircle, a triangle, or the like. In various embodiments, the polygonal shape of the cross-section is defined by a height h and a width b, wherein the moment of inertia of the shapeable member is preferably defined by at least 0.083 x b x h<3>.WO03041784 (A2)
US2008154207 (A1**)** discloses a splittable wire guide having a plurality of separable elongate components that may be separated or otherwise re-positioned relative to each other so as to alter the physical properties of the wire guide or to form a plurality of separate wire guide members.
US2016022215 (A1**)** is directed to intravascular devices, systems, and methods having a core wire with multiple flattened sections. In one aspect, a sensing guide wire is provided. The guide wire includes a first flexible elongate member; a sensing element positioned at a distal portion of the first flexible elongate member; and a second flexible elongate member coupled to the first flexible elongate member such that the second flexible elongate member extends distally from the first flexible elongate member; and wherein a distal portion of the first flexible elongate member includes at least two flattened sections, and wherein the first and second flexible elongate members are coupled along a portion of one of the at least two flattened sections.
US6290656 (B1**)** discloses a guide wire having a damped force vibration mechanism which isolates vibrations or shockwaves which otherwise would be transmitted along the length of the guide wire to its distal end. The damped force vibration mechanism helps absorb some of the energy created during the exchange of a delivery catheter or an interventional device on the guide wire or any shock or force generated from an external source. The damped force vibration mechanism absorbs vibrations or shock which would otherwise act on medical devices attached near the distal end of a guide wire. The damped force vibration mechanism may consist of a reduced segment on the guide wire which can be made from a material such as superelastic or plastically deformable, biocompatible materials. The damped force vibration mechanism can have a relatively straight configuration or can have a wave-shaped form, or other shape which helps absorb vibratory motion.

### Summary of Invention

### Technical Problem

An object of the present invention is to provide a guide wire that can achieve both excellent torque transmission performance and excellent shaping property.

### Solution to Problem

This object is achieved by the guide wire described in claim 1.

Further, in the guide wire of the present invention, in the plan view of the flat plate, it is preferable that the proximal portion of the first plate-shaped portion and the distal portion of the second plate-shaped portion are in contact with each other or overlap each other.

Further, in the guide wire of the present invention, it is preferable that at least the width of the proximal portion of the first plate-shaped portion gradually decreases toward a proximal end direction.

Further, in the guide wire of the present invention, the cross-sectional shape of the first linear portion and the cross-sectional shape of the second linear portion may be circular or semicircular, and the centers of the circle or the semicircle are preferably in a coaxial state positioned coaxially along the longitudinal direction of the wire.

In the guide wire of the present invention, it is preferable to include a coil which covers the first plate-shaped portion and the first linear portion and covers the second plate-shaped portion and the second linear portion.

### Advantageous Effects of Invention

According to the present invention, each of the first plate-shaped portion and the second plate-shaped portion, which are formed of separate members, serves as a part for shaping, and therefore shaping can be performed easily and reliably.

Also, since the first linear portion overlaps the second plate-shaped portion and the second linear portion overlaps the first plate-shaped portion, when torque is applied from the proximal side of the guide wire, the torque is reliably transmitted to the distal end of the guide wire.

Further, for example, when shaping is performed and used, it is possible to secure a shapeable range as long as possible.

In this manner, with the guide wire, it is possible to achieve both excellent torque transmission performance and shaping property.

### Brief Description of Drawings

[Fig. 1] Fig. 1 is a partial longitudinal cross-sectional plan view and auxiliary cross-sectional views of the first embodiment of a guide wire of the present invention.
[Fig. 2] Fig. 2 is an exploded plan view of a first core wire and a second core wire provided in the guide wire illustrated in Fig. 1.
[Fig. 3] Fig. 3 is a plan view and auxiliary cross-sectional views of the first core wire and the second core wire provided in a guide wire (a second embodiment) of the present invention.
[Fig. 4] Fig. 4 is a plan view and auxiliary cross-sectional views of the first core wire and the second core wire provided in a guide wire (a third embodiment) of the present invention.
[Fig. 5] Fig. 5 is a plan view of the first core wire and the second core wire provided in a guide wire (a fourth embodiment) of the present invention.
[Fig. 6] Fig. 6 is a plan view of the first core wire and the second core wire of a guide wire (a fifth embodiment) of the present invention.
[Fig. 7] Fig. 7 is an exploded plan view of the first core wire and the second core wire of a guide wire (a sixth embodiment) of the present invention.

### Description of Embodiments

Hereinafter, a guide wire of the present invention will be described in detail on the basis of preferred embodiments illustrated in the accompanying drawings.

### <First Embodiment>

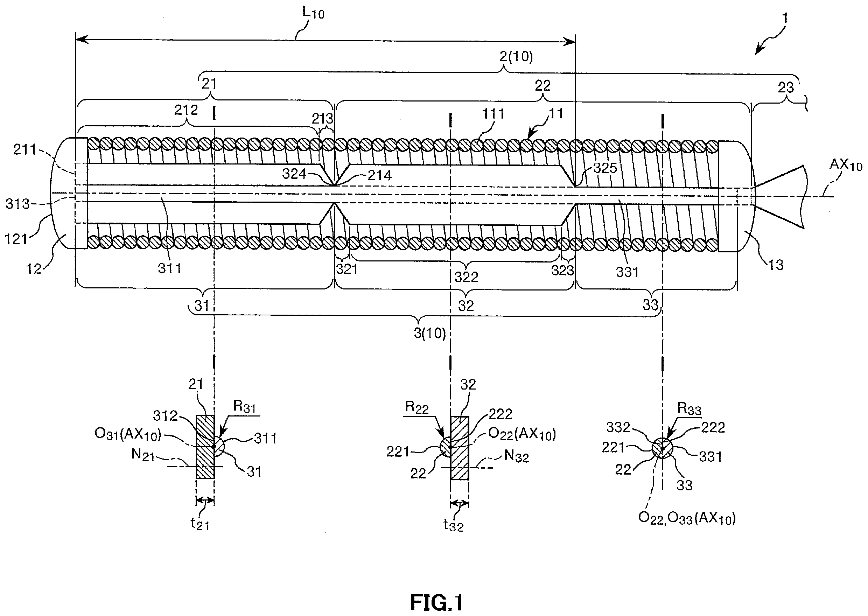

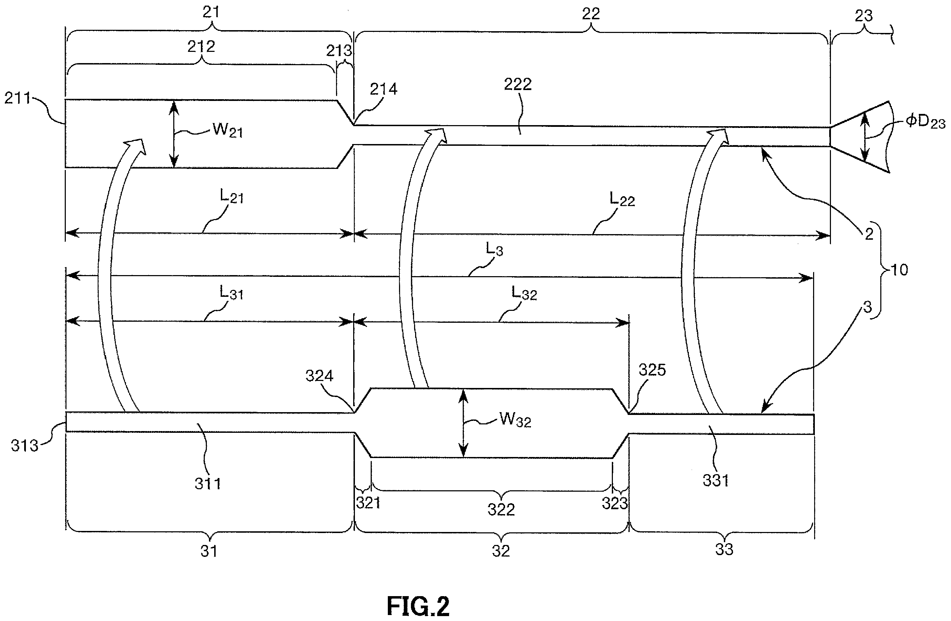

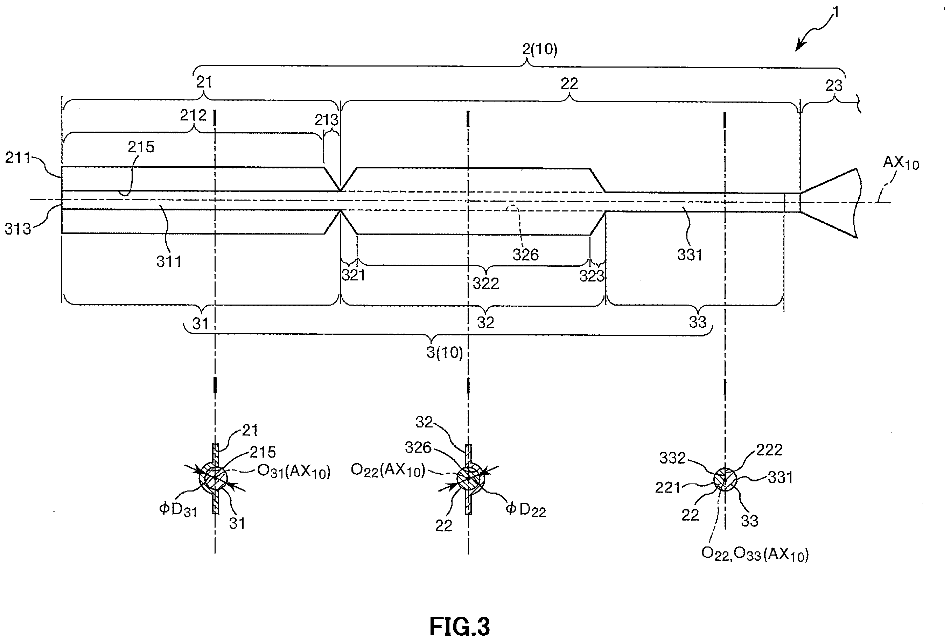

Fig. 1 shows a partial longitudinal cross-sectional plan view and auxiliary cross-sectional views illustrating a first embodiment of a guide wire of the present invention. Fig. 2 is an exploded plan view of a first core wire and a second core wire provided in the guide wire illustrated in Fig. 1. Note that, in the following description, a right side of Figs. 1 and 2 (the same applies to Figs. 3 to 7) is referred to as a "proximal end", and a left side is referred to as a "distal end" for convenience of description. Also, a longitudinal direction of the guide wire is shortened, a radial direction (a thickness direction) of the guide wire is schematically illustrated in an exaggerated manner, and a ratio between the longitudinal direction and the radial direction is different from an actual one in the drawings to facilitate an understanding of the invention.

A guide wire 1 illustrated in Fig. 1 is a catheter guide wire used by being inserted into a lumen of a catheter including an endoscope. The guide wire 1 includes a wire main body 10 including a first core wire 2 and a second core wire 3, and a spiral coil 11 which covers a portion of a distal side of the wire main body 10. Although an overall length of the guide wire 1 is not particularly limited, when the guide wire 1 is used for a PCI, for example, the overall length is preferably 200 mm or more and 5000 mm or less, and more preferably 1000 mm or more and 3000 mm or less.

As illustrated in Figs. 1 and 2, the first core wire 2 is made of an elongated body having flexibility. The first core wire 2 has a first plate-shaped portion 21, a first linear portion 22, and a tapered portion 23, which are disposed in order from the distal side thereof. Note that it is preferable that, for example, the outer diameter of the portion of the first core wire 2 on the proximal side from the tapered portion 23 is constant.

The first plate-shaped portion 21 is a portion having a plate shape. The first plate-shaped portion 21 is a flat plate in a natural state when no external force is applied. An overall length L₂₁ of the first plate-shaped portion 21 is not particularly limited, and is preferably, for example, 0.5 mm or more and 250.0 mm or less, and more preferably, 0.5 mm or more and 150 mm or less.

The first plate-shaped portion 21 has a constant width portion 212 and a gradually decreasing width portion 213. The constant width portion 212 is a portion in which a width W₂₁ from the distal portion of the first plate-shaped portion 21 to a predetermined length is constant along a longitudinal direction of the wire. The gradually decreasing width portion 213 is a portion in which the width W₂₁ gradually decreases toward the proximal side at the proximal portion of the first plate-shaped portion 21. The width W₂₁ of the constant width portion 212 is not particularly limited, and is preferably, for example, 0.080 mm or more and 0.880 mm or less, and more preferably 0.100 mm or more and 0.350 mm or less. Note that a gradually decreasing width portion in which the width W₂₁ gradually decreases toward the distal side may be formed at the distal portion of the first plate-shaped portion 21.

Further, a thickness t₂₁ of the first plate-shaped portion 21 is constant along the longitudinal direction of the wire. The thickness t₂₁ is not particularly limited, and is preferably, for example, 0.015 mm or more and 0.070 mm or less. Note that although the thickness t₂₁ is constant along the longitudinal direction of the wire in this embodiment, the thickness t₂₁ is not limited thereto, and may, for example, gradually decrease toward the distal side.

Such a first plate-shaped portion 21 can be bent and deformed into a desired shape together with a second linear portion 31 of the second core wire 3 overlapping the first plate-shaped portion 21, and can be used for shaping. The shaping is called "reshaping". By the shaping, it is possible to easily and reliably select the traveling direction of the guide wire 1 in the living body, and thus, the operability of the guide wire 1 is remarkably improved.

The first linear portion 22 is provided continuously on the proximal side of the first plate-shaped portion 21. The first linear portion 22 is a portion having a linear shape that is thinner than the first plate-shaped portion 21. In the present embodiment, the overall length L₂₂ of the first linear portion 22 is longer than the overall length L₂₁ of the first plate-shaped portion 21.

As illustrated in Fig. 1, the cross-sectional shape of the first linear portion 22 is semicircular. A radius R₂₂ of the first linear portion 22 is constant along the longitudinal direction of the wire. The radius R₂₂ is not particularly limited, and is, for example, preferably the same as the thickness t₂₁.

The tapered portion 23 is provided continuously on the proximal side of the first linear portion 22. The tapered portion 23 is a portion in which an outer diameter φD₂₃ gradually decreases toward the distal side. Therefore, the rigidity of the first core wire 2 can be gradually reduced toward the distal side by the tapered portion 23, and for example, the followability of the guide wire 1 in the living body is improved.

As illustrated in Figs. 1 and 2, the second core wire 3 is an elongated body having flexibility which is configured separately from the first core wire 2, and is shorter than the first core wire 2. An overall length L₃ of the second core wire 3 is preferably equal to or shorter than the total sum of the overall length L₂₁ and the overall length L₂₂ of the first core wire 2.

The distal portion of the second core wire 3 is fixed to the distal portion of the first core wire 2 via a fixing material 12, and the proximal portion thereof is fixed to the vicinity of a boundary portion between the first linear portion 22 and the tapered portion 23 of the first core wire 2 via a fixing material 13. Note that it is preferable that each of the fixing material 12 and the fixing material 13 is made of a solder (brazing material), but it is not limited thereto, and for example, an adhesive agent may be used. Further, a distal end 121 of the fixing material 12 is preferably rounded in order to prevent in vivo damage.

The second core wire 3 has a second linear portion 31, a second plate-shaped portion 32, and a third linear portion 33 disposed in order from the distal side thereof.

The second plate-shaped portion 32 has a plate shape, and is disposed to overlap the first linear portion 22 of the first core wire 2, that is, to be in contact therewith. Note that an overall length L₃₂ of the second plate-shaped portion 32 is the same as the overall length L₂₁ of the first plate-shaped portion 21 in the present embodiment.

The second plate-shaped portion 32 is a portion which is bent and deformed into a desired shape together with the first linear portion 22 overlapping the second plate-shaped portion 32, and can be used for shaping in the same manner as the first plate-shaped portion 21. As described above, the first plate-shaped portion 21 and the second plate-shaped portion 32 of the guide wire 1, which are made of separate members, are portions responsible for shaping, respectively.

As described above, the distal portion of the second core wire 3 is fixed to the first core wire 2 by the fixing material 12, and the proximal portion thereof is fixed to the first core wire 2 by the fixing material 13. As a result, an intermediate portion between the distal portion and the proximal portion of the second core wire 3 is not fixed to the first core wire 2, but is in a state of being in contact with the first core wire 2. Based on this configuration, when the distal portion of the guide wire 1 is bent, flexibility at the distal side of the guide wire 1 is obtained because the unfixed regions of the first core wire 2 and the second core wire 3 are bent independently as compared with a case where the overall second core wire 3 is fixed to the first core wire 2 by bonding. This improves the degree of freedom of shaping. Further, the abutting resistance of the guide wire 1 can also be reduced. The "abutting resistance" is the hardness when the distal end of the guide wire 1 abuts against the blood vessel wall. Further, when the abutting resistance is high, there is a risk of piercing of the blood vessel wall with the guide wire 1. However, in contrast, as the abutting resistance is low, it is possible to prevent or suppress the risk.

On the other hand, the first core wire 2 and the second core wire 3 may be fixed to each other at arbitrary positions. The rigidity or the torque transmission performance of the guide wire 1 can be arbitrarily changed, by changing the place where the fixation is performed or the number of fixing points . For example, in addition to the fixation using the fixing material 12 and the fixing material 13, when fixing the entire third linear portion 33 to the first core wire 2, the torque transmission performance is improved. As another fixing aspect, for example, there are fixation at the boundary portion between the first plate-shaped portion 21 and the second plate-shaped portion 32, fixation of the first plate-shaped portion 21 of the first core wire 2 and the second linear portion 31 of the second core wire 3, fixation of the first linear portion 22 of the first core wire 2 and the second plate-shaped portion 32 of the second core wire 3, and the like. By appropriately selecting the fixations, it is possible to provide a highly flexible guide wire 1, a guide wire 1 having a high torque transmission performance, or a guide wire 1 having excellent shaping property, depending on various operations. Examples of the fixation include a method of welding, a method of adhesion using solder (brazing filler metal) or an adhesive, and the like.

The second plate-shaped portion 32 is a flat plate in a natural state before being shaped similar to the first plate-shaped portion 21. Further, a normal line N₂₁ of the first plate-shaped portion 21 and a normal line N₃₂ of the second plate-shaped portion 32 in the natural state are directed in the same direction. That is, the thickness direction of the first plate-shaped portion 21 and the thickness direction of the second plate-shaped portion 32 are directed in the same direction. Therefore, for example, the operation of superimposing the first core wire 2 and the second core wire 3 at the time of manufacturing the guide wire 1 becomes easy. Additionally, the two-dimensional shaping of the guide wire 1 can be easily performed. The shaping of the guide wire 1 is performed by bending the guide wire 1 in the normal direction of the first plate-shaped portion 21 or the second plate-shaped portion 32. Accordingly, when the normal line N₂₁ of the first plate-shaped portion 21 and the normal line N₃₂ of the second plate-shaped portion 32 are directed in the same direction, the bending directions of the first plate-shaped portion 21 and the second plate-shaped portion 32 can be made identical to each other. This is preferable in performing the two-dimensional shaping, for example, in a case where the first bent portion and the second bent portion are provided on the guide wire 1 to perform the shaping of the two-stage shape, since it is possible to provide the first bent portion and the second bent portion on the same plane including the long axis of the guide wire 1.

The cross-sectional shape of the first linear portion 22 is semicircular as described above. Therefore, the first linear portion 22 has a side surface 221 curved in a semicircular arc shape, and a flat side surface 222. Further, by making the side surface 222 among the side surface 221 and the side surface 222 face the second plate-shaped portion 32, the first linear portion 22 and the second plate-shaped portion 32 enter a state of stably overlapping each other.

Further, the second plate-shaped portion 32 has a gradually decreasing width portion 321 having a width W₃₂ gradually decreasing toward the distal side formed at the distal portion, and a gradually decreasing width portion 323 having the width W₃₂ gradually decreasing toward the proximal side formed at the proximal portion. An intermediate portion between the gradually decreasing width portion 321 and the gradually decreasing width portion 323 is a constant width portion 322 in which the width W₃₂ is constant along the longitudinal direction of the wire. In the present embodiment, the width W₃₂ at the constant width portion 322 is the same as the width W₂₁ of the first plate-shaped portion 21, but it is not limited thereto, and the width W₃₂ may be greater than the width W₂₁.

A thickness t₃₂ of the second plate-shaped portion 32 is constant along the longitudinal direction of the wire, similarly to the thickness t₂₁ of the first plate-shaped portion 21. In the present embodiment, the thickness t₃₂ is the same as the thickness t₂₁ of the first plate-shaped portion 21, but it is not limited thereto, and the thickness t₃₂ may be thinner than the thickness t₂₁. Further, the thickness t₃₂ is not limited to being constant along the longitudinal direction of the wire, but may, for example, gradually decrease toward the distal direction.

The overall length L₃₂, the width W₃₂ and the thickness t₃₂ have the above-described sizes, and the overall length L₂₁, the width W₂₁ and the thickness t₂₁ also have the above-mentioned sizes. By appropriately combining the respective sizes, it is possible to obtain the guide wire 1 having different degrees of shaping, depending on various operations.

As illustrated in Fig. 1, a distal end 324 (distal portion) of the second plate-shaped portion 32 is in contact with a proximal end 214 (proximal portion) of the adjacent first plate-shaped portion 21 on the distal side thereof in a plan view. As a result, the second plate-shaped portion 32 is in a state of being disposed on the distal side as much as possible. In such an arrangement, the first plate-shaped portion 21 and the second plate-shaped portion 32 form apparently continuous plate-shaped portions. Therefore, it is possible to secure a long shapeable region of the guide wire 1. Generally, the shaping is preferably performed at the distal portion of the guide wire 1. Therefore, when disposing the second plate-shaped portion 32 on the distal side as much as possible, a state preferable for shaping is obtained.

In addition, the gradually decreasing width portion 321 formed on the distal side of the second plate-shaped portion 32 and the gradually decreasing width portion 213 formed on the proximal side of the first plate-shaped portion 21 can work together to reliably prevent the end portions of the second plate-shaped portion 32 and the first plate-shaped portion 21 from biting each other, for example, when the shaping is performed on the second plate-shaped portion 32. Therefore, it is possible to prevent the second plate-shaped portion 32 and the first plate-shaped portion 21 from being undesirably shaped.

The length L₁₀ from the distal end 211 of the first plate-shaped portion 21 to the proximal end 325 of the second plate-shaped portion 32 along the longitudinal direction of the wire is preferably 0.5 mm or more and 250 mm or less, more preferably 0.5 mm or more and 100 mm or less, and even more preferably 0.5 mm or more and 50 mm or less. This contributes to achieving both the torque transmission performance and the shaping property.

The second linear portion 31 is provided continuously on the distal side of the second plate-shaped portion 32. The second linear portion 31 is a portion having a linear shape that is thinner than the second plate-shaped portion 32. The second linear portion 31 is disposed to overlap the first plate-shaped portion 21, that is, to be in contact with the first plate-shaped portion 21, on the same side as the side on which the second plate-shaped portion 32 overlaps the first linear portion 22.

In the present embodiment, the overall length L₃₁ of the second linear portion 31 is the same as the overall length L₂₁ of the first plate-shaped portion 21, but the present invention is not limited thereto, and the overall length L₃₁ may be, for example, shorter than the overall length L₂₁. When the overall length L₃₁ is shorter than the overall length L₂₁, a distal end 313 of the second linear portion 31 is fixed in the middle of the first plate-shaped portion 21 in the longitudinal direction.

As illustrated in Fig. 1, the cross-sectional shape of the second linear portion 31 is semicircular. Therefore, the second linear portion 31 has a side surface 311 curved in a semicircular arc shape, and a flat side surface 312. By making the side surface 312 among the side surface 311 and the side surface 312 face the first plate-shaped portion 21, the first plate-shaped portion 21 and the second linear portion 31 enter a state of stably overlapping each other. Note that a radius R₃₁ of the second linear portion 31 is constant along the longitudinal direction of the wire. The radius R₃₁ is not particularly limited, and may be, for example, the same as the thickness t₃₂.

The third linear portion 33 is provided continuously on the proximal side of the second plate-shaped portion 32. The third linear portion 33 is a portion having a linear shape that is thinner than the second plate-shaped portion 32. The third linear portion 33 is disposed to overlap the first linear portion 22 together with the second plate-shaped portion 32, that is, to be in contact with the first linear portion 22. By disposing the third linear portion 33, the second plate-shaped portion 32 enters a state of being separated from the fixing material 13. Therefore, it is possible to prevent or suppress the influence of the fixation by the fixing material 13 on the second plate-shaped portion 32 at the time of shaping the second plate-shaped portion 32. Further, the third linear portion 33 contributes to reliably transmitting the torque, which is applied from the proximal side of the guide wire 1, to the second core wire 3. Furthermore, the torque applied from the proximal side of the guide wire 1 is transmitted to the first core wire 2 and the second core wire 3 without biasing since the third linear portion 33 is disposed to overlap the first linear portion 22.

As illustrated in Fig. 1, the cross-sectional shape of the third linear portion 33 is semicircular. Accordingly, the third linear portion 33 has a side surface 331 curved in a semicircular arc shape, and a flat side surface 332. Further, by making the side surface 332 among the side surface 331 and the side surface 332 face the side surface 222 of the first linear portion 22, the third linear portion 33 and the first linear portion 22 enter a state of stably overlapping each other. Note that a radius R₃₃ of the third linear portion 33 is constant along the longitudinal direction of the wire. The radius R₃₃ is not particularly limited, and is preferably, for example, the same as the radius R₂₂.

As described above, the cross-sectional shape of the first linear portion 22 and the cross-sectional shape of the second linear portion 31 each have a semicircular shape. A center O₂₂ of the semicircle in the first linear portion 22 and a center O₃₁ of the semicircle in the second linear portion 31 are in a coaxial state positioned on the same one axis AX₁₀ along the longitudinal direction of the wire. In other words, the center O₂₂ and the center O₃₁ overlap each other when viewed from the distal side in the natural state. A center O₃₃ of the semicircle in the third linear portion 33 and the center O₂₂ of the semicircle in the first linear portion 22 are also in a coaxial state positioned on the axis AX₁₀. In this case, a "circle" is formed by the cross-sectional shape of the third linear portion 33 and the cross-sectional shape of the first linear portion 22.

In combination of the respective coaxial states and the fact that the first linear portion 22 overlaps the second plate-shaped portion 32 and the third linear portion 33 and that the second linear portion 31 overlaps the first plate-shaped portion 21, when the torque is applied from the proximal side of the guide wire 1, the torque is reliably transmitted to the distal end of the guide wire 1.

The guide wire 1 described above can achieve both excellent torque transmission performance and excellent shaping property.

Further, since the wire main body 10 of the guide wire 1 is formed by making the first core wire 2 and the second core wire 3 overlap each other, the breaking strength is improved.

The constituent materials of the first core wire 2 and the second core wire 3 as described above are not particularly limited, and it is possible to use various metal materials such as alloys exhibiting pseudoelasticity including superelastic alloys, such as stainless steel, piano wire, cobalt type alloy, and nickel titanium alloy. When a superelastic alloy is used as the constituent material of the first core wire 2 and the second core wire 3, a treatment for eliminating superelasticity of a portion relating to shaping may be performed in order to improve the shaping property. The shaping portion means the first plate-shaped portion 21 and the first linear portion 22 of the first core wire 2 and the entire second core wire 3, that is, from the second linear portion 31 of the second core wire 3 to the third linear portion 33. As a treatment for eliminating the superelasticity, it is possible to use a known method such as heat treatment and cold working. Further, the first core wire 2 and the second core wire 3 may be made of different materials, but it is preferable that they are made of the same or the same kind of material.

The outer diameters of the linear portions of the first core wire 2 and the second core wire 3 are not particularly limited and can be outer diameters suitable as a guide wire. The outer diameters of the linear portions of the first core wire 2 and the second core wire 3 are, for example, 40 µm to 360 µm.

The coil 11 covers the first plate-shaped portion 21 and the first linear portion 22 of the first core wire 2, and covers the entire second core wire 3, that is, from the second linear portion 31 of the second core wire 3 to the third linear portion 33. Like the second core wire 3, the coil 11 is fixed to the first core wire 2 via the fixing material 12 and the fixing material 13. By installing such a coil 11, for example, the contact area of the surface of the wire main body 10 in a living body is reduced, and the sliding resistance can be reduced. As a result, the operability of the guide wire 1 is further improved.

Note that the coil 11 is formed by winding an element wire 111 around the wire main body 10, and the element wires 111 adjacent to each other in the longitudinal direction of the wire are in contact with each other in a natural state, namely, a so-called close winding state. The guide wire 1 is not limited to such a close winding configuration, and may have a rough winding portion in which the adjacent element wires 111 are spaced apart from each other.

Further, in the guide wire 1, it is preferable that at least the second plate-shaped portion 32 is spaced apart from the coil 11, regardless of whether or not the shaping is performed. Therefore, the degree of freedom of shaping in the second plate-shaped portion 32 can be sufficiently secured.

In the guide wire 1, it is preferable that at least the outer peripheral portion of the coil 11 is coated with a hydrophilic material. As a result, the hydrophilic material is wetted to produce lubricity, the sliding resistance of the guide wire 1 is reduced, and the slidability is improved. Therefore, the operability of the guide wire 1 is improved. As the hydrophilic material, for example, it is possible to use a cellulose-based polymer substance, a polyethylene oxide-based polymer substance, a maleic anhydride-based polymer substance (for example, a maleic anhydride copolymer such as methyl vinyl ether-maleic anhydride copolymer), an acrylamide-based polymer material (for example, a polyacrylamide, a polyglycidyl methacrylate-dimethyl acrylamide (PGMA-DMAA) block copolymer), water-soluble nylon, polyvinyl alcohol, polyvinyl pyrrolidone, and the like.

### <Second Embodiment>

Fig. 3 shows a plan view and auxiliary cross-sectional views of a first core wire and a second core wire included in a guide wire (the second embodiment) of the present invention.

Hereinafter, the second embodiment of the guide wire of the present invention will be described with reference to the drawing, but the differences from the above-described embodiment will be mainly described, and the description of the same matter will not be provided.

The present embodiment is the same as the first embodiment except that it has a positioning portion that maintains the coaxial state.

As illustrated in Fig. 3, in the present embodiment, the cross-sectional shape of the portion of the first linear portion 22 overlapping the second plate-shaped portion 32 is circular. The cross-sectional shape of the second linear portion 31 is also circular. It is more preferable that an outer diameter φD₂₂ of the first linear portion 22 having a circular shape and an outer diameter φD₃₁ of the second linear portion 31 have the same size.

In the first plate-shaped portion 21, a first groove 215 is formed along the longitudinal direction of the wire. The first groove 215 is formed by bending and plastically deforming the first plate-shaped portion 21 by, for example, a pressing machine. Then, the second linear portion 31 can be inserted into the first groove 215.

A second groove 326 is formed in the second-plate shaped portion 32 along the longitudinal direction of the wire. Like the first groove 215, the second groove 326 is also formed by bending and plastically deforming the second plate-shaped portion 32 by, for example, a pressing machine. The first linear portion 22 can then be inserted into the second groove 326.

By the insertion as described above, the center O₂₂ of the circle in the first linear portion 22 and the center O₃₁ of the circle in the second linear portion 31 are in a coaxial state positioned on the axis AX₁₀, and the coaxial state can be reliably maintained. As a result, it is possible to reliably improve torque transmission performance.

In the guide wire 1 of the present embodiment, the first groove 215 and the second groove 326 function as positioning portions that maintain the coaxial state.

### <Third Embodiment>

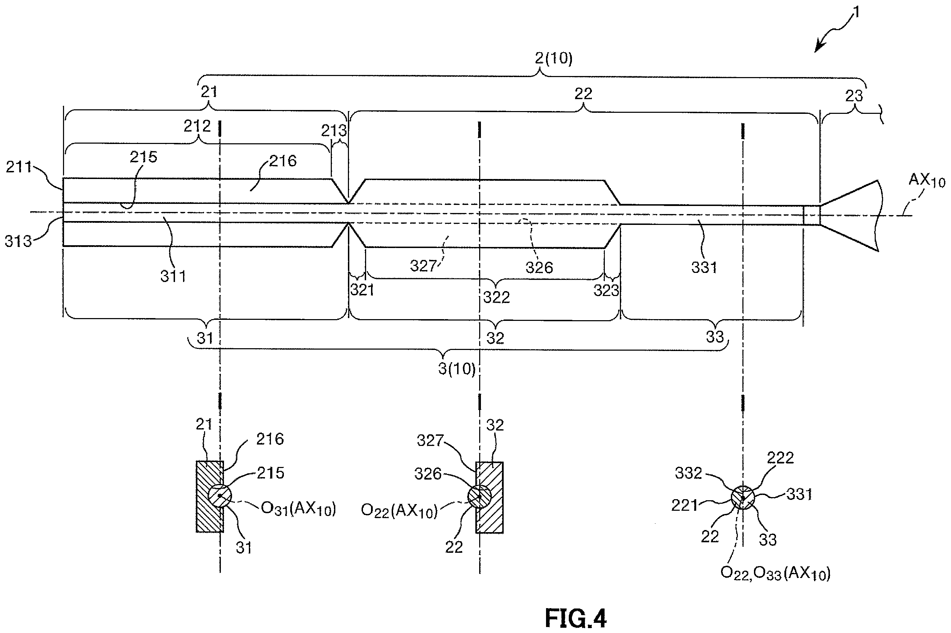

Fig. 4 depicts a plan view and auxiliary cross-sectional views of a first core wire and a second core wire included in a guide wire (a third embodiment) of the present invention.

Hereinafter, the third embodiment of the guide wire of the present invention will be described with reference to the drawing, but the differences from the above-described embodiment will be mainly described, and the description of the same matter will not be provided.

This embodiment is the same as the first embodiment except that the method of forming the first groove and the second groove is different.

In the present embodiment illustrated in Fig. 4, the first groove 215 is formed by cutting a surface 216 of the first plate-shaped portion 21 facing the second linear portion 31 side by, for example, a cutting machine. As a result, since the first groove 215 can be provided as a groove having a size large enough to fit the second linear portion 31, the second linear portion 31 is prevented from being detached from the first groove 215.

Further, the second groove 326 is formed by cutting a surface 327 of the second plate-shaped portion 32 facing the first linear portion 22 side by, for example, a cutting machine. Accordingly, since the second groove 326 can be provided as a groove having a size large enough to fit the first linear portion 22, the first linear portion 22 is prevented from being detached from the second groove 326.

### <Fourth Embodiment>

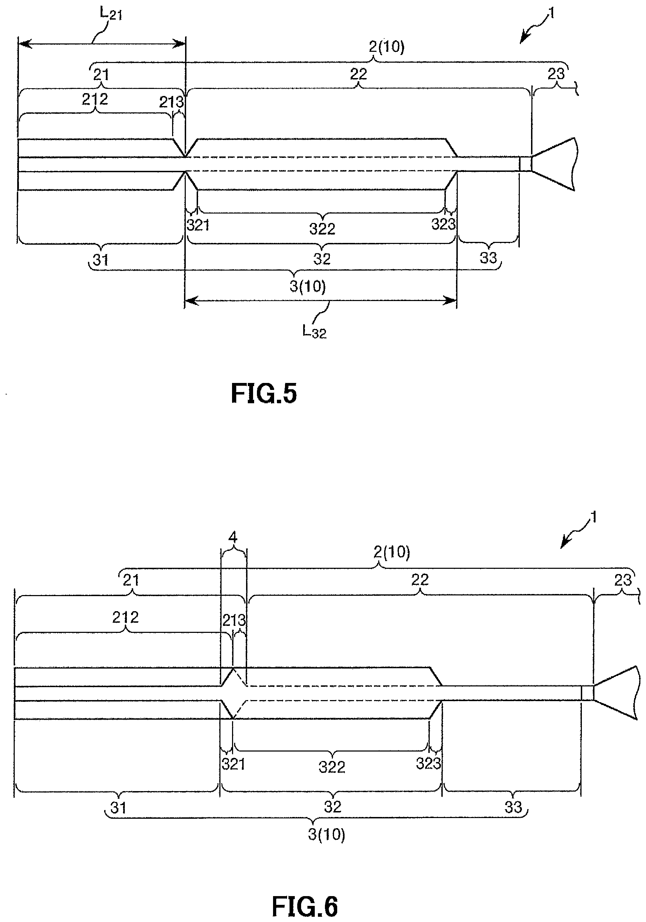

Fig. 5 is a plan view of a first core wire and a second core wire of a guide wire (a fourth embodiment) of the present invention.

Hereinafter, the fourth embodiment of the guide wire of the present invention will be described with reference to the drawing, but the differences from the above-described embodiment will be mainly described, and the description of the same matter will not be provided.

This embodiment is the same as the first embodiment except that a magnitude relation between the overall length of the first plate-shaped portion and the overall length of the second plate-shaped portion is different.

In the present embodiment shown in Fig. 5, the overall length L₃₂ of the second plate-shaped portion 32 is longer than the overall length L₂₁ of the first plate-shaped portion 21. This makes it easier to perform the shaping on the second plate-shaped portion 32.

### <Fifth Embodiment>

Fig. 6 is a plan view of a first core wire and a second core wire of a guide wire (a fifth embodiment) of the present invention.

Hereinafter, the fifth embodiment of the guide wire of the present invention will be described with reference to the drawing, but the differences from the above-described embodiment will be mainly described, and the description of the same matter will not be provided.

This embodiment is the same as the first embodiment except that a positional relation between the first plate-shaped portion and the second plate-shaped portion is different.

As shown in Fig. 6, in this embodiment, an overlapping portion 4 is formed in which a proximal portion of the first plate-shaped portion 21 and the distal portion of a second plate-shaped portion 32 overlap in a plan view. In the overlapping portion 4, torque is smoothly transmitted from the second plate-shaped portion 32 to the first plate-shaped portion 21. Therefore, the torque transmission performance is improved.

### <Sixth Embodiment>

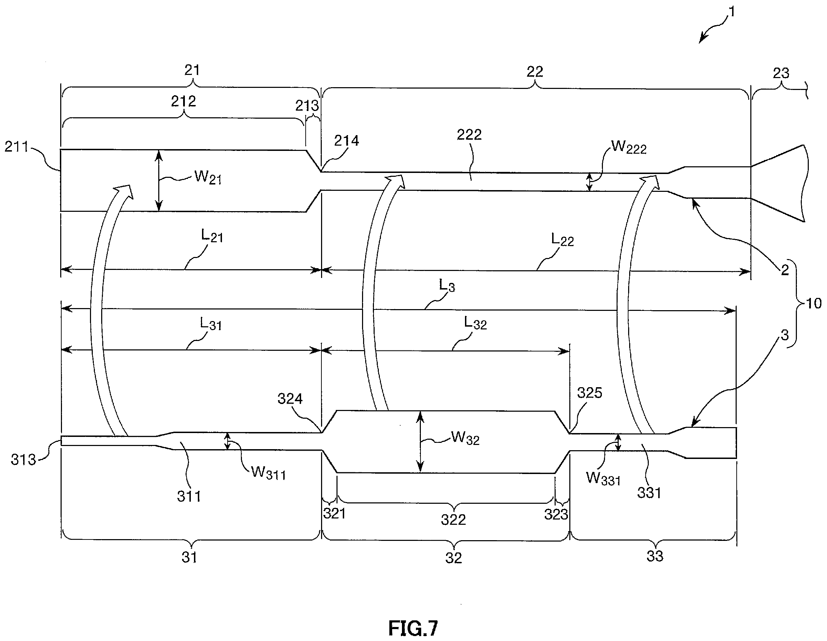

Fig. 7 is an exploded plan view of a first core wire and a second core wire of a guide wire (a sixth embodiment) of the present invention.

Hereinafter, the sixth embodiment of the guide wire of the present invention will be described with reference to the drawing, but the differences from the above-described embodiment will be mainly described, and the description of the same matter will not be provided.

This embodiment is the same as the first embodiment except that the shapes of the first core wire and the second core wire are different from each other.

In the present embodiment shown in Fig. 7, a width W₂₂₂ of the side surface 222 of the first linear portion 22 of the first core wire 2 decreases stepwise toward the distal side. In the third linear portion 33 of the second core wire 3, a width W₃₃₁ of the side surface 331 also decreases stepwise toward the distal side, corresponding to the shape of the first linear portion 22. Note that it is preferable that the cross-sectional shape of the first linear portion 22 and the cross-sectional shape of the third linear portion 33 together form a "circle".

In addition, a width W₃₁₁ of the side surface 311 of the second linear portion 31 of the second core wire 3 also decreases stepwise toward the distal side.

The guide wire 1 thus gradually decreases in rigidity toward the distal side as a whole by having the shape as described above. As a result, the guide wire 1 has, for example, good flexibility at the distal portion, and the followability to the blood vessel or the like and safety are improved.

Note that the width W₂₂₂, the width W₃₁₁, and the width W₃₃₁ decrease stepwise toward the distal side as described above, and the number of stages is one in the illustrated configuration, but is not limited to this and may be plural stages.

Each of the width W₂₂₂, the width W₃₁₁, and the width W₃₃₁ may, for example, continuously decrease toward the distal side, in addition to the stepwise decrease toward the distal side.

Although the guide wire has been described with reference to the embodiment illustrated in the drawings, the present invention is not limited thereto, and each part constituting the guide wire may be substituted for any configuration capable of exerting the same function. Also, arbitrary components may be added.

Further, the guide wire of the present invention may be a combination of arbitrary two or more configurations (features) among the above embodiments.

Although the number of the second core wires is one in each of the above embodiments, it is not limited thereto, and may be, for example, plural. For example, in a case where the number of second core wires is two, the guide wire can be one in which the first core wire is disposed between the two second core wires.

Although a magnitude relation between the overall length of the first core wire and the overall length of the second core wire is set such that the overall length of the first core wire is longer than the overall length of the second core wire in each of the above embodiments, it is not limited thereto, and the overall length of the second core wire may be longer than the overall length of the first core wire.

Further, the first core wire may further include a linear portion having a liner shape that is provided continuously on the distal side of the first plate-shaped portion and is thinner than the first plate-shaped portion.

Further, in the first core wire and the second core wire, the gradually decreasing width portion can be omitted.

Further, in the second core wire, the third linear portion can be omitted. In this case, it is preferable that the cross-sectional shape of the portion of the first linear portion of the first core wire overlapping the third linear portion is circular.

### Industrial Applicability

The guide wire of the present invention is a guide wire including a flexible first core wire, and a flexible second core wire configured separately from the first core wire. The first core wire has a first plate-shaped portion having a plate shape, and a first linear portion provided continuously to the proximal side of the first plate-shaped portion and having a linear shape thinner than the first plate-shaped portion. The second core wire has a second plate-shaped portion having a plate shape and disposed to overlap the first linear portion, and a second linear portion that is provided continuously on the distal side of the second plate-shaped portion, has a linear shape thinner than the second plate-shaped portion, and is disposed to overlap the first plate-shaped portion. Therefore, it is possible to achieve excellent compatibility between the torque transmission performance and the shaping property. Therefore, the guide wire of the present invention has industrial applicability.

### Reference Signs List

- 1: guide wire
- 2: first core wire
- 21: first plate-shaped portion
- 211: distal end
- 212: constant width portion
- 213: gradually decreasing width portion
- 214: proximal end
- 215: first groove
- 216: surface
- 22: first linear portion
- 221, 222: side surface
- 23: tapered portion
- 3: second core wire
- 31: second linear portion
- 311, 312: side surface
- 313: distal end
- 32: second plate-shaped portion
- 321: gradually decreasing width portion
- 322: constant width portion
- 323: gradually decreasing width portion
- 324: distal end
- 325: proximal end
- 326: second groove
- 327: surface
- 33: third linear portion
- 331, 332: side surface
- 4: overlapping portion
- 10: wire main body
- 11: coil
- 111: element wire
- 12: fixing material
- 121: distal end
- 13: fixing material
- AX₁₀: axis line
- φD₂₂, φD₂₃, φD₃₁: outer diameter
- L₁₀: length
- L₂₁, L₂₂, L₃, L₃₁, L₃₂: overall length
- N₂₁, N₃₂: normal line
- O₂₂, O₃₁, O₃₃: center
- R₂₂, R₃₁, R₃₃: radius
- t₂₁, t₃₂: thickness
- W₂₁, W_{222,} W₃₁₁, W₃₂, W₃₃₁: width

## Claims

1. A guide wire (1) comprising a flexible first core wire (2), and a flexible second core wire (3) configured separately from the first core wire (2), wherein the first core wire (2) has a first plate-shaped portion (21) having a plate shape, and a first linear portion (22) provided continuously on a proximal side of the first plate-shaped portion (21) and having a linear shape thinner than the first plate-shaped portion (21), and the second core wire (3) has a second plate-shaped portion (32) having a plate shape and disposed to overlap and be in contact with the first linear portion (22), and a second linear portion (31) that is provided continuously on a distal side of the second plate-shaped portion (32), has a linear shape thinner than the second plate-shaped portion (32), and is disposed to overlap and be in contact with the first plate-shaped portion (21).

2. The guide wire (1) according to claim 1, wherein the second core wire (3) is shorter than the first core wire (2), and at least a distal portion and a proximal portion of the second core wire (3) are fixed with respect to the first core wire (2).

3. The guide wire (1) according to claim 1 or 2, wherein a length (L₁₀) from a distal end of the first plate-shaped portion (21) to a proximal end of the second plate-shaped portion (32) along a longitudinal direction of the wire is 0.5 mm or more and 250 mm or less.

4. The guide wire (1) according to any one of claims 1 to 3, wherein the first plate-shaped portion (21) and the second plate-shaped portion (32) are flat plates in a natural state in which no external force is applied, and a normal line (N₂₁) of the first plate-shaped portion (21) and a normal line (N₃₂) of the second plate-shaped portion (32) are directed in the same direction.

5. The guide wire (1) according to any one of claims 1 to 4, wherein a center of a cross-sectional shape of the first linear portion (22) and a center of a cross-sectional shape of the second linear portion (31) are in a coaxial state positioned coaxially along the longitudinal direction of the wire (1).

6. The guide wire (1) according to claim 5, further comprising: a positioning portion which maintains the coaxial state.

7. The guide wire (1) according to claim 6, wherein the positioning portion includes a first groove (215) which is formed in the first plate-shaped portion (21) and into which the second linear portion (31) is inserted, and a second groove (326) which is formed in the second plate-shaped portion (32) and into which the first linear portion (22) is inserted.

8. The guide wire (1) according to any one of claims 1 to 7, wherein the cross-sectional shape of the first linear portion (22) and the cross-sectional shape of the second linear portion (31) have the same shape.

9. The guide wire (1) according to any one of claims 1 to 8, wherein the cross-sectional shape of the first linear portion (22) and the cross-sectional shape of the second linear portion (31) are circular or semicircular.

10. The guide wire (1) according to any one of claims 1 to 9, wherein the second core wire (3) has a third linear portion (33) that is provided continuously on the proximal side of the second plate-shaped portion (32), has a linear shape thinner than the second plate-shaped portion (32), and is disposed to overlap the first linear portion (22).

11. The guide wire (1) according to any one of claims 1 to 10, wherein the overall length (L₃₂) of the second plate-shaped portion (32) is longer than the overall length (L₂₁) of the first plate-shaped portion (21).

12. The guide wire (1) according to any one of claims 1 to 10, wherein an overlapping portion (4) is formed in which a proximal portion of the first plate-shaped portion (21) and the distal portion of a second plate-shaped portion (32) overlap in a plan view.

13. The guide wire (1) according to claim 10, wherein
each of
- a width (W₂₂₂) of the side surface (222) of the first linear portion (22) of the first core wire (2), and
- in the third linear portion (33) of the second core wire (3), a width (W₃₃₁) of the side surface (331), and
- a width (W₃₁₁) of the side surface (311) of the second linear portion (31) of the second core wire (3)
decreases stepwise in one or plural stages or continuously toward the distal side of the guide wire (1).

## Patentansprüche

1. Führungsdraht (1), der einen flexiblen ersten Kerndraht (2) und einen flexiblen zweiten Kerndraht (3) umfasst, der getrennt von dem ersten Kerndraht (2) ausgebildet ist, wobei der erste Kerndraht (2) einen ersten plattenförmigen Abschnitt (21), der eine Plattenform aufweist, und einen ersten linearen Abschnitt (22) aufweist, der durchgehend auf einer proximalen Seite des ersten plattenförmigen Abschnitts (21) vorgesehen ist, und eine lineare Form aufweist, die dünner als der erste plattenförmige Abschnitt (21) ist, und der zweite Kerndraht (3) einen zweiten plattenförmigen Abschnitt (32), der eine Plattenform aufweist und so angeordnet ist, dass er den ersten linearen Abschnitt (22) überlappt und mit ihm in Kontakt steht, und einen zweiten linearen Abschnitt (31) aufweist, der durchgehend auf einer distalen Seite des zweiten plattenförmigen Abschnitts (32) vorgesehen ist, welcher eine lineare Form aufweist, die dünner als der zweite plattenförmige Abschnitt (32) ist, und so angeordnet ist, dass er den ersten plattenförmigen Abschnitt (21) überlappt und mit ihm in Kontakt steht.

2. Führungsdraht (1) nach Anspruch 1, wobei der zweite Kerndraht (3) kürzer ist als der erste Kerndraht (2), und mindestens ein distaler Abschnitt und ein proximaler Abschnitt des zweiten Kerndrahtes (3) in Bezug auf den ersten Kerndraht (2) fixiert sind.

3. Führungsdraht (1) nach Anspruch 1 oder 2, wobei eine Länge (L₁₀) von einem distalen Ende des ersten plattenförmigen Abschnitts (21) zu einem proximalen Ende des zweiten plattenförmigen Abschnitts (32) entlang einer Längsrichtung des Drahtes 0, 5 mm oder mehr und 250 mm oder weniger beträgt.

4. Führungsdraht (1) nach einem der Ansprüche 1 bis 3, wobei der erste plattenförmige Abschnitt (21) und der zweite plattenförmige Abschnitt (32) flache Platten in einem natürlichen Zustand sind, in dem keine äußere Kraft aufgebracht wird, und eine Normallinie (N₂₁) des ersten plattenförmigen Abschnitts (21) und eine Normallinie (N₃₂) des zweiten plattenförmigen Abschnitts (32) in dieselbe Richtung gerichtet sind.

5. Führungsdraht (1) nach einem der Ansprüche 1 bis 4, wobei ein Zentrum einer Querschnittsform des ersten linearen Abschnitts (22) und ein Zentrum einer Querschnittsform des zweiten linearen Abschnitts (31) in einem koaxialen Zustand koaxial entlang der Längsrichtung des Drahtes (1) positioniert sind.

6. Führungsdraht (1) nach Anspruch 5, ferner umfassend: einen Positionierungsabschnitt, der den koaxialen Zustand aufrechthält.

7. Führungsdraht (1) nach Anspruch 6, wobei der Positionierungsabschnitt eine erste Nut (215), die in dem ersten plattenförmigen Abschnitt (21) ausgebildet ist und in die der zweite lineare Abschnitt (31) eingesetzt ist, und eine zweite Nut (326) aufweist, die in dem zweiten plattenförmigen Abschnitt (32) ausgebildet ist und in die der erste lineare Abschnitt (22) eingesetzt ist.

8. Führungsdraht (1) nach einem der Ansprüche 1 bis 7, wobei die Querschnittsform des ersten linearen Abschnitts (22) und die Querschnittsform des zweiten linearen Abschnitts (31) die gleiche Form aufweisen.

9. Führungsdraht (1) nach einem der Ansprüche 1 bis 8, wobei die Querschnittsform des ersten linearen Abschnitts (22) und die Querschnittsform des zweiten linearen Abschnitts (31) kreisförmig oder halbkreisförmig sind.

10. Führungsdraht (1) nach einem der Ansprüche 1 bis 9, wobei der zweite Kerndraht (3) einen dritten linearen Abschnitt (33) aufweist, der durchgehend auf der proximalen Seite des zweiten plattenförmigen Abschnitts (32) vorgesehen ist, eine lineare Form aufweist, die dünner ist als der zweite plattenförmige Abschnitt (32), und so angeordnet ist, dass er den ersten linearen Abschnitt (22) überlappt.

11. Führungsdraht (1) nach einem der Ansprüche 1 bis 10, wobei die Gesamtlänge (L₃₂) des zweiten plattenförmigen Abschnitts (32) länger ist als die Gesamtlänge (L₂₁) des ersten plattenförmigen Abschnitts (21).

12. Führungsdraht (1) nach einem der Ansprüche 1 bis 10, wobei ein Überlappungsabschnitt (4) ausgebildet ist, in dem sich ein proximaler Abschnitt des ersten plattenförmigen Abschnitts (21) und der distale Abschnitt eines zweiten plattenförmigen Abschnitts (32) in einer Draufsicht überlappen.

13. Führungsdraht (1) nach Anspruch 10, wobei jede von
- einer Breite (W₂₂₂) der Seitenfläche (222) von dem ersten linearen Abschnitt (22) des ersten Kerndrahtes (2), und
- in dem dritten linearen Abschnitte (33) des zweiten Kerndrahtes (3), einer Breite (W₃₃₁) der Seitenfläche (331), und
- einer Breite (W₃₁₁) der Seitenfläche (311) von dem zweiten linearen Abschnitt (31) des zweiten Kerndrahtes (3),
schrittweise in einer oder mehreren Stufen oder kontinuierlich in Richtung der distalen Seite des Führungsdrahtes (1) abnimmt.

## Revendications

1. Fil (1) guide comprenant un premier fil central (2) flexible, et un deuxième fil central (3) flexible configuré séparément du premier fil central (2), dans lequel le premier fil central (2) comporte une première partie en forme de plaque (21) présentant une forme de plaque, et une première partie linéaire (22) ménagée en continu sur un côté proximal de la première partie en forme de plaque (21) et présentant une forme linéaire plus mince que la première partie en forme de plaque (21), et le deuxième fil central (3) comporte une deuxième partie en forme de plaque (32) présentant une forme de plaque et disposée pour chevaucher la première partie linéaire (22) et être en contact avec celle-ci, et une deuxième partie linéaire (31) qui est ménagée en continu sur un côté distal de la deuxième partie en forme de plaque (32), présente une forme linéaire plus mince que la deuxième partie en forme de plaque (32), et est disposée pour chevaucher la première partie en forme de plaque (21) et être en contact avec celle-ci.

2. Fil (1) guide selon la revendication 1, dans lequel le deuxième fil central (3) est plus court que le premier fil central (2), et au moins une partie distale et une partie proximale du deuxième fil central (3) sont fixées par rapport au premier fil central (2).

3. Fil (1) guide selon la revendication 1 ou 2, dans lequel une longueur (L₁₀) allant d'une extrémité distale de la première partie en forme de plaque (21) à une extrémité proximale de la deuxième partie en forme de plaque (32) le long d'une direction longitudinale du fil est de 0,5 mm ou plus et de 250 mm ou moins.

4. Fil (1) guide selon l'une quelconque des revendications 1 à 3, dans lequel la première partie en forme de plaque (21) et la deuxième partie en forme de plaque (32) sont des plaques plates dans un état naturel dans lequel aucune force externe n'est appliquée, et une ligne normale (N₂₁) de la première partie en forme de plaque (21) et une ligne normale (N₃₂) de la deuxième partie en forme de plaque (32) sont dirigées dans la même direction.

5. Fil (1) guide selon l'une quelconque des revendications 1 à 4, dans lequel un centre d'une forme en coupe transversale de la première partie linéaire (22) et un centre d'une forme en coupe transversale de la deuxième partie linéaire (31) sont dans un état coaxial positionné coaxialement le long de la direction longitudinale du fil (1).

6. Fil (1) guide selon la revendication 5, comprenant en outre : une partie de positionnement qui maintient l'état coaxial.

7. Fil (1) guide selon la revendication 6, dans lequel la partie de positionnement comporte une première rainure (215) qui est formée dans la première partie en forme de plaque (21) et dans laquelle la deuxième partie linéaire (31) est insérée, et une deuxième rainure (326) qui est formée dans la deuxième partie en forme de plaque (32) et dans laquelle la première partie linéaire (22) est insérée.

8. Fil (1) guide selon l'une quelconque des revendications 1 à 7, dans lequel la forme en coupe transversale de la première partie linéaire (22) et la forme en coupe transversale de la deuxième partie linéaire (31) présentent la même forme.

9. Fil (1) guide selon l'une quelconque des revendications 1 à 8, dans lequel la forme en coupe transversale de la première partie linéaire (22) et la forme en coupe transversale de la deuxième partie linéaire (31) sont circulaires ou semi-circulaires.

10. Fil (1) guide selon l'une quelconque des revendications 1 à 9, dans lequel le deuxième fil central (3) comporte une troisième partie linéaire (33) qui est ménagée en continu sur le côté proximal de la deuxième partie en forme de plaque (32), présente une forme linéaire plus mince que la deuxième partie en forme de plaque (32) et est disposée pour chevaucher la première partie linéaire (22).

11. Fil (1) guide selon l'une quelconque des revendications 1 à 10, dans lequel la longueur globale (L₃₂) de la deuxième partie en forme de plaque (32) est plus longue que la longueur globale (L₂₁) de la première partie en forme de plaque (21).

12. Fil (1) guide selon l'une quelconque des revendications 1 à 10, dans lequel une partie de chevauchement (4) est formée dans laquelle une partie proximale de la première partie en forme de plaque (21) et la partie distale d'une deuxième partie en forme de plaque (32) se chevauchent sur une vue en plan.

13. Fil (1) guide selon la revendication 10, dans lequel chacune parmi
- une largeur (W₂₂₂) de la surface latérale (222) de la première partie linéaire (22) du premier fil central (2), et
- dans la troisième partie linéaire (33) du deuxième fil central (3), une largeur (W₃₃₁) de la surface latérale (331), et
- une largeur (W₃₁₁) de la surface latérale (311) de la deuxième partie linéaire (31) du deuxième fil central (3) diminue progressivement d'un ou plusieurs niveaux ou en continu vers le côté distal du fil (1) guide.
